(19) Europäisches Patentamt
European Patent Office
Office européen des brevets

(11) Veröffentlichungsnummer: **0 005 162**
**B1**

(12) # EUROPÄISCHE PATENTSCHRIFT

(45) Veröffentlichungstag der Patentschrift: **30.12.81**

(51) Int. Cl.³: **C 07 D 239/10**

(21) Anmeldenummer: **79100936.8**

(22) Anmeldetag: **29.03.79**

(54) **Verfahren zur Herstellung von 5,5-disubstituierten 4-Carbamido-hexahydropyrimidinen.**

(30) Priorität: **26.04.78 DE 2818157**

(43) Veröffentlichungstag der Anmeldung: **14.11.79 Patentblatt 79/23**

(45) Bekanntmachung des Hinweises auf die Patenterteilung: **30.12.81 Patentblatt 81/52**

(84) Benannte Vertragsstaaten: **BE CH DE FR GB IT**

(56) Entgegenhaltungen: **DE-A-1 670 261**

(73) Patentinhaber: **BASF Aktiengesellschaft, Carl-Bosch-Strasse 38, D-6700 Ludwigshafen (DE)**

(72) Erfinder: **Petersen, Harro, Dr., Kalmitstrasse 23, D-6710 Frankenthal (DE)**

**EP 0 005 162 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 5,5-disubstituierten 4-Carbamido-hexahydropyrimidinen

Die Erfindung betrifft ein Verfahren zur Herstellung von 5,5-disubstituierten 4-Carbamido-hexahydropyrimidinen durch Umsetzung von Hexahydropyrimidinen mit Carbamiden bei Temperaturen über 100°C.

Es ist bekannt, dass man durch Cyclokondensation von Harnstoffen mit einem Mol Formaldehyd und einem oder 2 Mol eines CH-aciden Aldehyds wie Isobutyraldehyd in Gegenwart von Säuren zu 4-Hydroxy-2-oxo-hexahydropyrimidinen gelangt, z.B.:

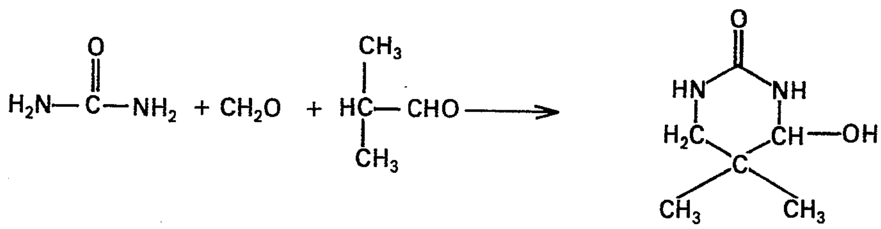

(Synthesis, Band 1973, Seiten 243 bis 292, insbesondere Seite 262 und 263). Diese Umsetzungen verlaufen mit befriedigenden Ausbeuten nur in den Fällen, in denen ein CH-acider Aldehyd eingesetzt wird, der in α-Stellung zwei Substituenten aufweist. Setzt man CH-acide Aldehyde ein, die in α-Stellung keinen (Acetaldehyd) oder nur einen Substituenten (n-Aldehyde) aufweisen, so erhält man bei der sauren Kondensation mit Harnstoffen 2-Oxo(thiono)-4-ureidohexahydropyrimidine (IV) oder Dioxo(dithiono)-decahydropyrimidine (V):

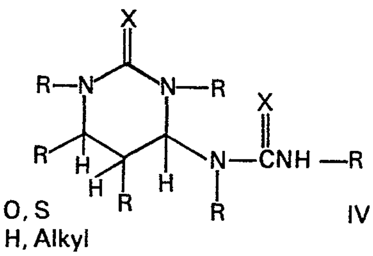

X = O, S
R = H, Alkyl

IV

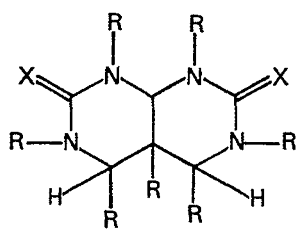

V

Bei Einsatz derartiger CH-acider Aldehyde bleibt die Reaktion nicht bei den 4-Hydroxy-Derivaten stehen (Synthesis Band 1973, Seiten 261 bis 280).

Ferner lassen sich 2-Oxo(thiono)-4-hydroxy-(alkoxy)-hexahydropyrimidine, die in 5-Stellung keinen oder nur einen Substituenten aufweisen, durch Umsetzung von Harnstoffen mit α,β-ungesättigten Aldehyden in Gegenwart von Säure herstellen, z.B.:

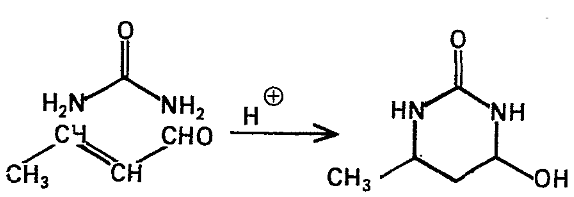

Die so hergestellten 4-Hydroxy-Derivate reagieren mit Harnstoffen zu den 4-Ureido-Derivaten (Synthesis, loc. cit., Seite 264). Die Synthese der in 5-Stellung unsubstituierten oder monosubstituierten 2-Oxo(thiono)-4-hydroxy-(alkoxy)-hexahydropyrimidine ist auf diesem Syntheseweg umständlich und verläuft mit unbefriedigenden Ausbeuten.

In den Monatsheften für Chemie, Band 96 (1965), Seiten 1950 bis 1966 (insbesondere 1957) wird beschrieben, dass die Substitution der Hydroxygruppe in 4-Stellung durch Nucleophile erschwert wird, wenn in 5-Stellung ein Substituent steht. Bei zwei Substituenten in 5-Stellung lässt sich eine Substitution der OH-Gruppe in Stellung 4 durch Harnstoffe unter den bisher bekannten Bedingungen nicht mehr durchführen. So ist es auch verständlich, dass es unter den üblichen Bedingungen der Säurekatalyse bisher nicht möglich war, in 5-Stellung disubstituierte 4-Hydroxyhexahydropyrimidine mit Harnstoffen zu 5,5-disubstituierten 2-Oxo(thiono)-4-ureido-hexahydropyrimidinen umzusetzen.

Aus den deutschen Offenlegungsschriften 1 670 261 und 1 670 262 geht hervor, dass man aliphatische N-Hexahydropyrimidyl-(4)-carbaminsäureester durch Kondensation von 4-Hydroxy-bzw. 4-Alkoxy-hexahydropyrimidinverbindungen mit aliphatischen Carbaminsäureestern bzw. Hydroxyalkylencarbamaten in Gegenwart einer Säure bei Temperaturen zwischen 0 bzw. 20 bis 100°C umsetzt. Die in diesen Offenlegungsschriften angegebenen Ausbeuten liegen zwischen 67 und maximal 88% der Theorie. Die Endstoffe müssen, gerade auch im industriellen Massstab, für die Weiterverarbeitung einer Reinigung unterzogen werden. Da die Kondensation in Gegenwart von Säuren durchgeführt wird, werden die Ausgangsstoffe durch diese Säuren teilweise

zersetzt. Die Neutralisation der Säure und die Abtrennung der dabei entstehenden Salze erschweren die Aufarbeitung. Die beiden Verfahren sind mit Bezug auf Ausbeute und Reinheit des Endstoffes sowie einfachen und wirtschaftlichen Betrieb unbefriedigend.

Es wurde nun gefunden, dass man 5,5-disubstituierte 4-Carbamidohexahydropyrimidine der Formel

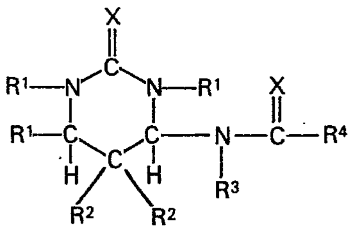

I,

worin die einzelnen Reste $R^1$, $R^2$ und $R^3$ gleich oder verschieden sein können und jeweils einen aliphatischen, cycloaliphatischen, araliphatischen oder aromatischen Rest bedeuten, darüber hinaus auch die Reste $R^1$ und $R^3$ jeweils ein Wasserstoffatom bezeichnen können, $R^4$ den Rest 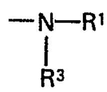

oder den Rest $-O-R^5$ bedeutet, $R^5$ einen aliphatischen Rest oder den Rest

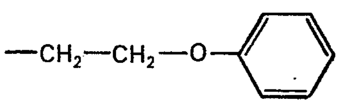

bezeichnet, die beiden Reste $R^3$, sofern $R^4$ den Rest 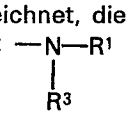

bezeichnet, auch zusammen mit der benachbarten Ureidogruppe für Glieder eines heterocyclischen Ringes stehen können, $R^3$, sofern $R^4$ den Rest $-OR^5$ bedeutet, und $R^4$ zusammen mit dem benachbarten Carbamidorest auch für Glieder eines heterocyclischen Ringes stehen können, die einzelnen Reste X gleich oder verschieden sein können und jeweils ein Sauerstoffatom oder ein Schwefelatom bedeuten, durch Umsetzung von Hexahydropyrimidinen der Formel

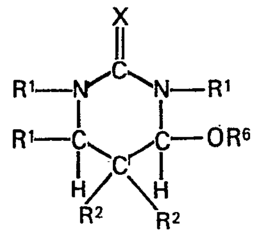

II,

worin $R^1$, $R^2$ und X die vorgenannte Bedeutung besitzen und $R^6$ ein Wasserstoffatom oder einen aliphatischen Rest bezeichnet, mit Carbamiden der Formel

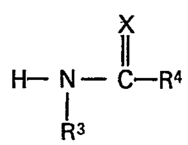

III,

worin $R^3$, $R^4$ und X die vorgenannte Bedeutung besitzen, vorteilhaft erhält, wenn man ohne Säurezusatz bei einer Temperatur zwischen 100 und 150°C umsetzt.

Die Umsetzung kann für den Fall der Verwendung von Harnstoff und von 2-Oxo-4-hydroxy-5,5-dimethyl-6-isopropyl-hexahydropyrimidin durch die folgenden Formeln wiedergegeben werden:

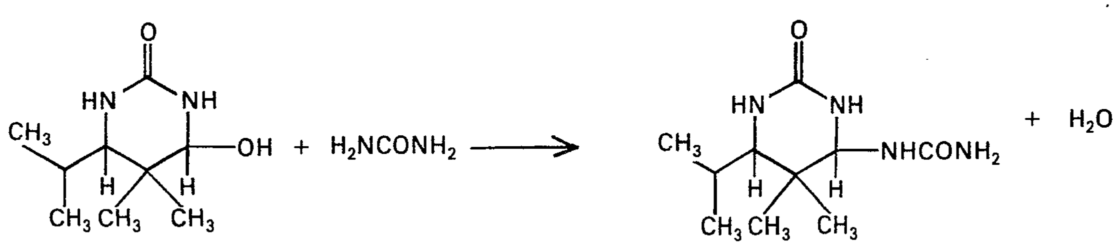

Die Umsetzung lässt sich für den Fall der Verwendung von 2-Oxo-4-hydroxy-5,5-dimethyl-6-isopropyl-hexahydropyrimidin und Carbaminsäureäthylester durch die folgenden Formeln wiedergeben:

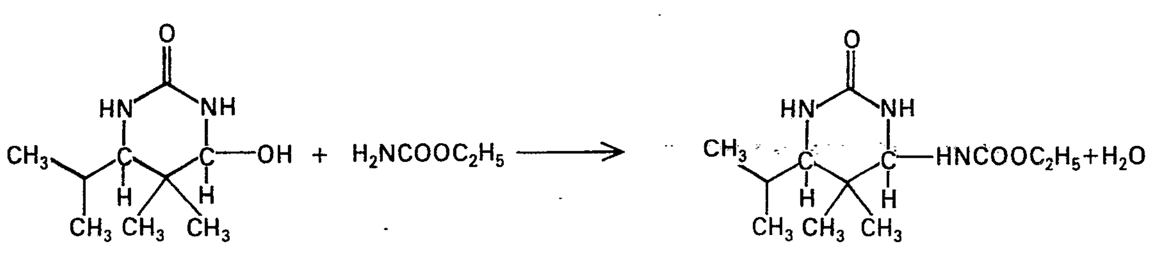

Im Hinblick auf den Stand der Technik liefert das Verfahren nach der Erfindung auf einfachem und wirtschaftlichem Wege eine grosse Zahl von bisher nicht beschriebenen 5,5-disubstituierten 4-Ureido-hexahydropyrimidinen in hoher Ausbeute und Reinheit sowie von neuen und bekannten 5,5-disubstituierten Hexahydropyrimidyl-4-carbaminsäureestern in besserer Ausbeute und Reinheit. Man braucht nicht in Gegenwart von Säure umzusetzen. Da keine Säurekatalyse benötigt wird, entfällt die Neutralisation und die Abtrennung von Salzen; Zersetzungsreaktionen der Ausgangs- und Endstoffe werden vermieden. Die nach dem erfindungsgemässen Verfahren herstellbaren Heterocyclen besitzen im Vergleich zu den in 5-Stellung unsubstituierten oder monosubstituierten Hexahydropyrimidinen eine sehr hohe Temperaturstabilität. Während die vorgenannten Stoffe bei Temperatureinwirkung von über 100°C leicht bis stark verfärbt werden, bleiben die erfindungsgemässen Endstoffe I praktisch farblos. Alle diese vorteilhaften Ergebnisse sind im Hinblick auf den Stand der Technik überraschend.

Die Ausgangsstoffe II und III können in stöchiometrischer Menge oder im Überschuss jeder Komponente zur anderen, vorzugsweise in einem Verhältnis von 1 bis 1,5, insbesondere 1 bis 1,1 Mol Ausgangsstoff III je Ausgangsstoff II, umgesetzt werden. Bevorzugte Ausgangsstoffe II und III und dementsprechend bevorzugte Endstoffe I sind solche, in deren Formeln die einzelnen Reste $R^1$, $R^2$ und $R^3$ gleich oder verschieden sein können und jeweils einen Alkylrest mit 1 bis 18, insbesondere mit 1 bis 7 Kohlenstoffatomen, einen Cyclohexylrest, einen Aralkylrest mit 7 bis 12 Kohlenstoffatomen oder einen Phenylrest bedeuten, darüber hinaus auch die Reste $R^1$ und $R^3$ jeweils ein Wasserstoffatom bezeichnen können, $R^4$ den Rest 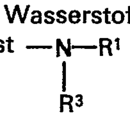

oder den Rest $-O-R^5$ bedeutet, die beiden Reste $R^3$, sofern $R^4$ den Rest 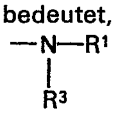

bezeichnet, auch zusammen mit der benachbarten Ureidogruppe für Glieder eines 5- oder 6-gliedrigen, heterocyclischen Ringes stehen können, $R^3$, sofern $R^4$ den Rest $-OR^5$ bedeutet, und $R^4$ zusammen mit dem benachbarten Carbamidorest auch für Glieder eines 5- oder 6-gliedrigen, heterocyclischen Ringes stehen können, die einzelnen Reste X gleich oder verschieden sein können und jeweils ein Sauerstoffatom oder ein Schwefelatom bedeuten, $R^6$ ein Wasserstoffatom oder ein Alkylrest mit 1 bis 18, insbesondere mit 1 bis 7 Kohlenstoffatomen bezeichnet, $R^5$ einen Alkylrest mit 1 bis 18, insbesondere 1 bis 7 Kohlenstoffatomen, den Rest $-(CH_2-CH_2-O)_n-R^6$, worin $R^6$ die vorgenannte allgemeine oder bevorzugte Bedeutung besitzt und n eine ganze Zahl, insbesondere von 1 bis 7, bedeutet, oder den Rest

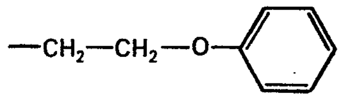

bezeichnet.

Die vorgenannten Reste und Ringe können noch durch unter den Reaktionsbedingungen inerte Gruppen, z.B. Alkylgruppen mit 1 bis 4 Kohlenstoffatomen, substituiert sein. Geeignete Harnstoffe III sind monosubstituierte, symmetrisch und asymmetrisch disubstituierte und trisubstituierte Harnstoffe.

So sind z.B. folgende Hexahydropyrimidine als Ausgangsstoffe II geeignet: 5,5-Dimethyl-, 5,5-Diäthyl-, 5,5-Di-n-propyl-, 5,5-Di-isopropyl-, 5,5-Di-n-butyl-, 5,5-Di-sek.-butyl-, 5,5-Diisobutyl-, 5,5-Di-tert.-butyl, 5-Phenyl-5-methyl, 5-Benzyl-5-methyl-, 5-Cyclohexyl-5-methyl-, 5-Methyl-5-isopropyl-4-hydroxy-2-oxohexahydropyrimidin; entsprechende, in 1-, 3- und/oder 6-Stellung durch die Methyl-, Äthyl-, n-Propyl-, Isopropyl-, n-Butyl-, Isobutyl-, sek.-Butyl-, tert.-Butyl-, Cyclohexyl-, Benzyl-, (2-Phenyl)-äthyl-(2)-, (2-Phenyl)-äthyl-(1)-, Phenyl-gruppe substituierte 4-Hydroxy-2-oxo-hexahydropyrimidine; homologe 4-Methoxy-, 4-Äthoxy-, 4-n-Propoxy-, 4-Isopropoxy-, 4-n-Butoxy-, 4-Isobutoxy-, 4-sek.-Butoxy-, 4-tert.-Butoxy-2-oxo-hexahydropyrimidine; entsprechende, in vorgenannten Stellungen unsubstituierte und/oder substituierte 2-Thionohexahydropyrimidine.

Geeignete Harnstoffe III sind beispielsweise: Harnstoff, Thioharnstoff, Monomethylharnstoff, Monophenylharnstoff, Monostearylharnstoff, symmetrischer Dimethylharnstoff, asymmetrischer Dimethylharnstoff, symmetrischer und asymmetrischer Diphenylharnstoff, Propylenharnstoff, Äthylenharnstoff, Monobenzyl-, Monocyclohexyl-, symmetrischer Dibenzyl-, asymmetrischer Dibenzyl-, symmetrischer und asymetrischer Dicyclohexyl-, Monoäthyl-, Monopropyl-, Monoisopropyl-, Monobutyl-, Mono-sek.-butyl-, Mono-tert.-butyl-, Mono-n-butylharnstoff; symmetrischer Diäthyl-, Dipropyl-, Diisopropyl-, Dibutyl-, Di-sek.-butyl-, Diisobutyl-, Di-tert.-butyl-harnstoff; asymmetrischer Diäthyl-, Dipropyl-, Diisopropyl-, Dibutyl-, Diisobutyl-, Di-sek.-butyl-, Di-tert.-butyl-harnstoff; Trimethyl-, Triäthyl-, Tripropyl-, Triisopropyl-, Tributyl-, Triisobutyl-, Tri-sek.-butyl-, Tri-tert.-butylharnstoff; entsprechend substituierte Thioharnstoffe.

Geeignete Carbamidsäureester III sind beispielsweise: Methyl-, Äthyl-, n-Propyl-, Isopropyl-, n-Butyl-, Isobutyl-, sek.-Butyl-, tert.-Butyl-, Cyclohexyl-, Benzyl-, (2-Phenyl)-äthyl-(2)-, (2-Phenyl)-äthyl-(1)-, Phenyl-ester der Carbaminsäure und entsprechende, am Stickstoffatom durch die Methyl-, Äthyl-, n-Propyl-, Isopropyl-, n-Butyl-, Isobutyl-, sek.-Butyl-, tert.-Butyl-, Cyclohexyl-, Benzyl-, (2-Phenyl)-äthyl-(2)-, (2-Phenyl)-äthyl-(1)-, Phenyl-gruppe substituierten Carbaminsäureester; 2-Oxo-oxazolidin, 2-Oxo-1,3-tetrahydrooxazin; Monoester von Äthylenglykol, Di-, Tri-, Tetra-, Penta-, Hexa-, Heptaäthylenglykol mit der Carbaminsäure und homologe, an der freien Hydroxylgruppe dieser Ester durch die Methyl-, Äthyl-, n-Propyl-, Isopropyl-, n-Butyl-, Isobutyl-, sek.-Butyl-, tert.-Butyl-gruppe verätherte Monoäther-mono-ester; Phenylglykolcarbaminsäuremonoester; entsprechende Thiocarbaminsäurederivate.

Die Umsetzung wird bei einer Temperatur zwischen 100 und 150°C, vorteilhaft zwischen 110 und 150°C, vorzugsweise von 121 bis 140°C, insbesondere von 125 bis 140°C, drucklos oder unter Druck, kontinuierlich oder diskontinuierlich durchgeführt. Man kann ohne Lösungsmittel umsetzen; zweckmässig verwendet man aber unter den Reaktionsbedingungen inerte Lösungsmittel, insbesondere mit Siedepunkten über 100°C. Bei Flüssigkeiten mit Siedepunkten unter 100°C wird die Reaktion in geschlossenen Gefässen unter Druck durchgeführt. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Dimethylformamid; und entsprechende Gemische. Bevorzugte Lösungsmittel sind Toluol und Xylole. Es ist nicht erforderlich, dass die Ausgangsstoffe und Endstoffe in diesen Lösungsmitteln löslich sind. Die Unlöslichkeit der Endstoffe ermöglicht eine besonders leichte Aufarbeitung. Ferner muss das gebildete Reaktionswasser nicht unbedingt abgeführt werden. Es ist jedoch vorteilhaft, das Wasser durch azeotrope Destillation abzutrennen. Zweckmässig verwendet man das Lösungsmittel in einer Menge von 400 bis 10 000 Gewichtsprozent, vorzugsweise von 400 bis 1000 Gewichtsprozent, bezogen auf Ausgangsstoff II. Die Umsetzung kann zweckmässig in Abwesenheit von Lösungsmitteln durchgeführt werden, wenn eine oder beide Ausgangskomponenten oder der Endstoff flüssig oder bei der Reaktionstemperatur schmelzbar sind.

Gegebenenfalls kann man, im allgemeinen nur im Falle der Umsetzung mit Harnstoffen III, Basen, z.B. in Gestalt von Natronlauge, zweckmässig in katalytischen Mengen, bevorzugt in Mengen von 0,005 bis 0,01 Äquivalenten Base je Ausgangsstoff II, zusetzen.

Die Reaktion kann wie folgt durchgeführt werden: Ein Gemisch der Ausgangsstoffe II und III und zweckmässig Lösungsmittel wird während 0,25 bis 3 Stunden bei der Reaktionstemperatur gehalten. Dann wird der Endstoff aus dem Gemisch in üblicher Weise, z.B. durch Kristallisation und Filtration, abgetrennt.

Die nach dem Verfahren der Erfindung herstellbaren 5,5-disubstituierten 4-Carbamido-hexahydropyrimidine sind wertvolle Ausgangsstoffe für die Herstellung von Farbstoffen, Schädlingsbekämpfungsmitteln und Hilfsmitteln in der Textilchemie, Lackharzchemie, Holzwerkstoffindustrie. Ihre N-Methylol- und N-Alkoxymethylverbindungen sind als reaktive Vernetzer in der Textilchemie, Lackharzchemie, Holzwerkstoffindustrie vorteilhaft verwendbar. So kann z.B. ein Baumwollgewebe mit einem Quadratmetergewicht von 125 g mit einer wässrigen Flotte, die 200 Teile N,N'-Dimethylol-2-oxo-5,5-dimethylhexahydropyrimidyl-4(N)-carbaminsäureäthylester und 20 Teile Magnesiumchloridhexahydrat im Liter enthält, imprägniert, getrocknet, bei 160°C in einem Kondensationsaggregat 5 Minuten behandelt und so knitterfrei ausgerüstet werden. Es werden Trockenknitterwinkel von 248° (Kette + Schuss) sowie Nassknitterwinkel von 304° (Kette + Schuss) erhalten. Die Ausrüstungseffekte sind nicht nur gegen mehrere Chlorwäschen und Maschinenkochwäschen, sondern auch gegen eine saure Hygiene-Nachbehandlung beständig.

Die in den Beispielen genannten Teile bedeuten Gewichtsteile.

Beispiel 1

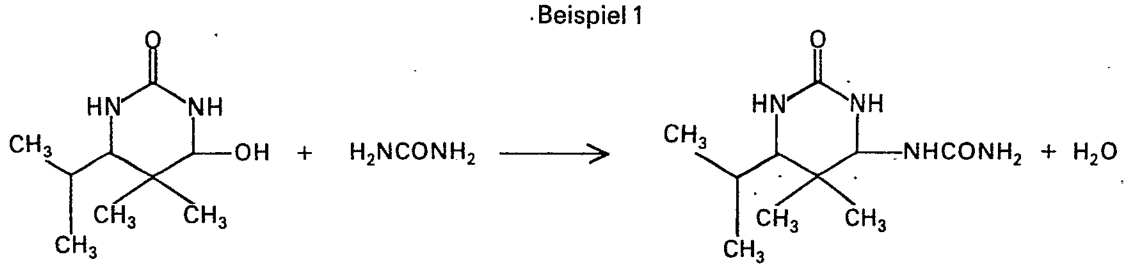

In einer Rührapparatur mit Rührer, Wasserabscheider, Rückflusskühler und Heizung werden 930 Teile 2-Oxo-4-hydroxy-5,5-dimethyl-6-isopropyl-hexahydropyrimidin zusammen mit 300 Teilen Harnstoff und 3000 Teilen Xylol auf 130°C erwärmt. Bei Siedetemperatur (130 bis 135°C) werden in 40 Minuten 90 Teile Wasser aus dem Reaktionsgemisch durch azeotrope Destillation über den Wasserabscheider ausgekreist. Der Endstoff wird nach dem Abkühlen durch Filtration abgetrennt. Nach Trocknung werden 1110 Teile 2-Oxo-4-ureido-5,5-dimethyl-6-isopropylhexahydropyrimidin in kristalliner Form erhalten. Das entspricht einer Ausbeute von 97,4% der Theorie. Schmelzpunkt (aus Wasser) 208 bis 209°C.

Beispiel 2

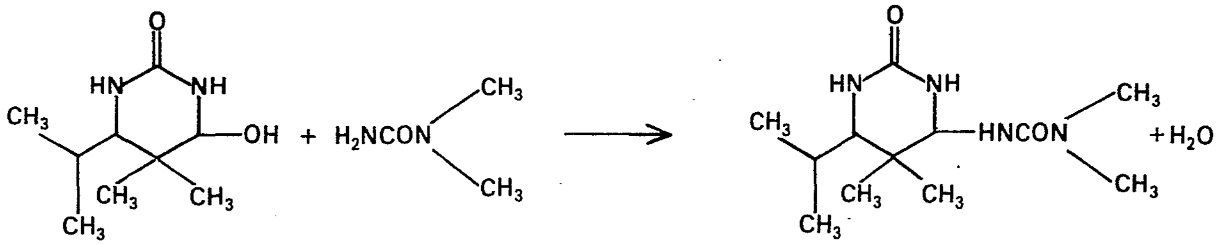

93 Teile 2-Oxo-4-hydroxy-5,5-dimethyl-6-isopropyl-hexahydropyrimidin, 44 Teile asymmetrischer Dimethylharnstoff (in 350 Teilen Xylol) und 350 Teile Xylol werden in der in Beispiel 1 beschriebenen Apparatur eine Stunde bei Rückflusstemperatur (130 bis 140°C) erwärmt. 9 Teile Wasser werden ausgekreist; es entsteht eine klare Lösung. Nach dem Abkühlen kristallisiert der Endstoff aus. Es werden 124 Teile 2-Oxo-4-(dimethyl-ureido)-5,5-dimethyl-6-isopropyl-hexahydropyrimidin erhalten. Das entspricht einer Ausbeute von 97% der Theorie. Fp (aus Methanol) 160°C.

Beispiel 3

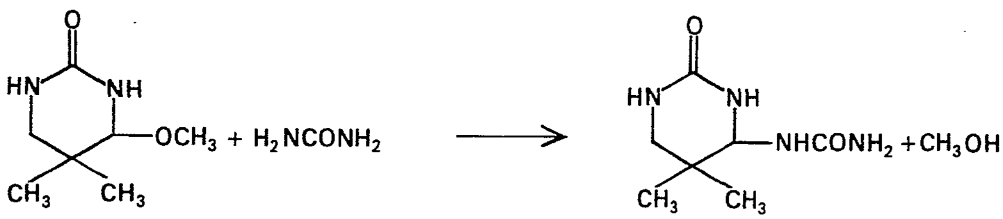

Analog Beispiel 1 werden 79 Teile 2-Oxo-4-methoxy-5,5-dimethyl-hexahydropyrimidin, 30 Teile Harnstoff und 300 Teile Xylol unter Rühren auf 120°C erhitzt. Nach einer Gesamtreaktionsdauer von 30 Minuten werden 15 Teile Methanol in der Vorlage aufgefangen. Nach Abkühlen auf Raumtemperatur wird der Endstoff abfiltriert und getrocknet. Es werden 89 Teile 2-Oxo-4-ureido-5,5-dimethyl-hexahydropyrimidin erhalten. Das entspricht einer Ausbeute von 96% der Theorie. Schmelzpunkt (Wasser/Äthanol 1:1) 243°C.

Beispiel 4

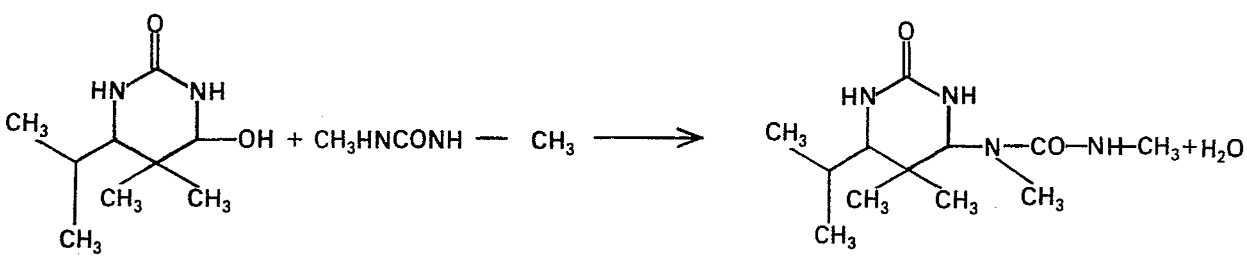

Analog Beispiel 1 werden 93 Teile 2-Oxo-4-hydroxy-5,5-dimethyl-6-isopropyl-hexahydropyrimidin zusammen mit 44 Teilen symmetrischem Dimethylharnstoff und 350 Teilen Xylol 90 Minuten auf Rückflusstemperatur (135°C) erwärmt. Hierbei werden 8,9 Teile Wasser ausgekreist. Nach Abkühlen wird der Endstoff abfiltriert und getrocknet. Es werden 122 Teile 2-Oxo-4-(N-Methyl-N'-methyl-ureido)-5,5-dimethyl-6-isopropyl-hexahydropyrimidin erhalten. Das entspricht einer Ausbeute von 95,3% der Theorie. Fp (Methanol) 211°C.

Beispiel 5

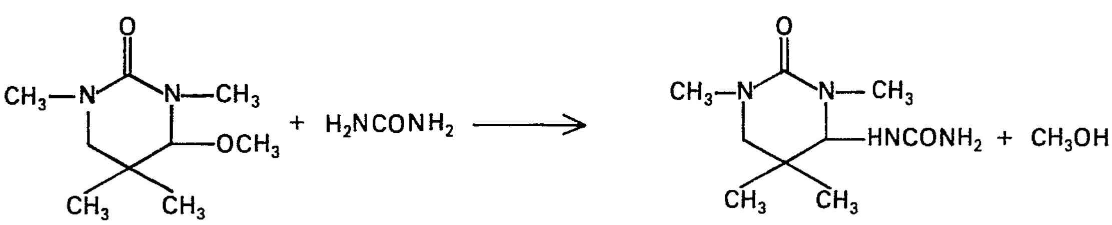

37,2 Teile 2-Oxo-4-methoxy-1,3,5,5-tetramethyl-hexyhydropyrimidin, 12 Teile Harnstoff und 200 Teile Xylol werden analog Beispiel 1 bei 126°C gehalten. In einem Zeitraum von 35 Minuten wird

Methanol abgespalten und in einer Vorlage abgefangen. Nach Abkühlen und 2-stündigem Stehen bei Raumtemperatur wird der Endstoff abfiltriert und getrocknet. Es werden 39,5 Teile 2-Oxo-4-ureido-1,3,5,5-tetramethyl-hexahydropyrimidin erhalten. Das entspricht einer Ausbeute von 92,3% der Theorie. Fp 192°C (unter Zersetzung).

Beispiel 6

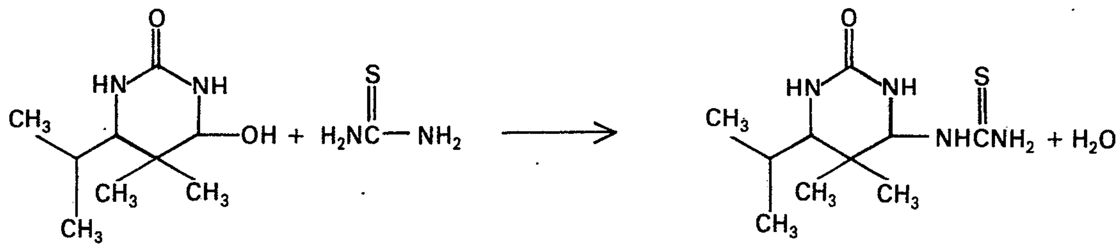

Analog Beispiel 1 wird das Gemisch aus 93 Teilen 2-Oxo-4-hydroxy-5,5-dimethyl-6-isopropyl-hexahydropyrimidin, 38 Teilen Thioharnstoff und 700 Teilen Xylol auf Rückflusstemperatur (130°C) erhitzt. Innerhalb von 20 Minuten werden 8,9 Teile Wasser ausgekreist. Nach Abtrennen des kristallinen Reaktionsproduktes und Trocknen werden 119 Teile 2-Oxo-4-thioureido-5,5-dimethyl-6-isopropyl-hexahydropyrimidin erhalten. Das entspricht einer Ausbeute von 97,5% der Theorie. Fp (Äthanol) 136°C.

Beispiel 7

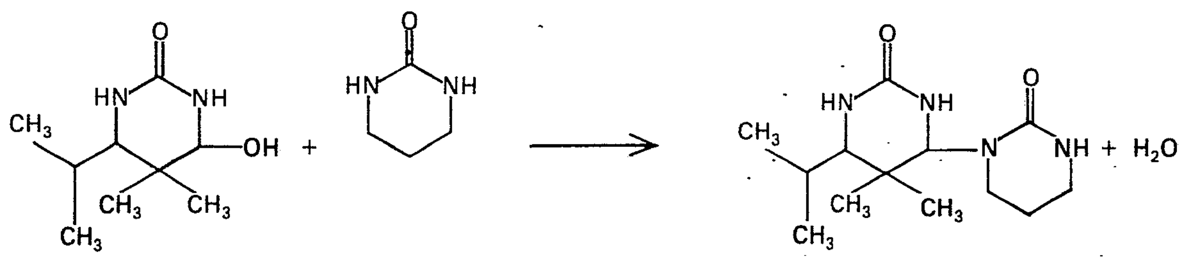

Analog Beispiel 1 werden 93 Teile 2-Oxo-4-hydroxy-5,5-dimethyl-6-isopropyl-hexahydropyrimidin zusammen mit 50 Teilen Propylenharnstoff in 700 Teilen Xylol auf Rückflusstemperatur (130°C) erhitzt. Im Verlauf von 3 Stunden werden 9 Teile Wasser ausgekreist. Nach Abtrennen und Trocknen werden 126 Teile 2-Oxo-4-(2'-oxo-hexahydropyrimidyl-(N)-5,5-dimethyl-6-isopropyl-hexahydropyrimidin erhalten. Das entspricht einer Ausbeute von 94% der Theorie. Fp 264°C.

Beispiel 8

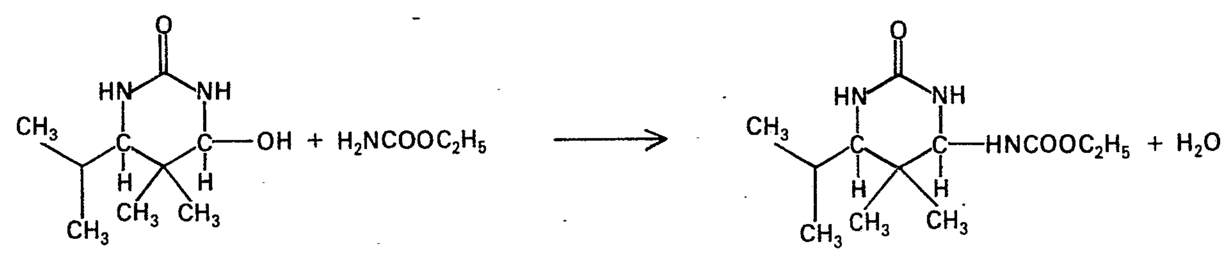

In einer Rührapparatur mit Rührer, Wasserabscheider, Rückflusskühler und Heizung werden 93 Teile 2-Oxo-4-hydroxy-5,5-dimethyl-6-isopropyl-hexahydropyrimidin zusammen mit 44,5 Teilen Äthylurethan und 500 Teilen Xylol eine halbe Stunde auf 130°C am Rückfluss erwärmt, wobei das gebildete Wasser aus dem Reaktionsgemisch durch azeotrope Destillation über den Wasserabscheider abgetrennt wird. Anschliessend werden 200 Teile Xylol abdestilliert. Nach Abkühlen wird der kristalline Endstoff abfiltriert und getrocknet. Es werden 123 Teile 2-Oxo-5,5-dimethyl-6-isopropyl-hexahydropyrimidyl-4-N-carbaminsäureäthylester erhalten. Das entspricht einer Ausbeute von 96% der Theorie vom Fp 200°C (Aceton).

Beispiel 9

93 Teile 2-Oxo-4-hydroxy-5,5-dimethyl-6-isopropylhexahydropyrimidin, 59,5 Teile Methoxyäthylcarbamat und 400 Teile Xylol werden analog Beispiel 8 30 Minuten bei Rückflusstemperatur (135°C) erwärmt, wobei das dabei entstehende Reaktionswasser über den Wasserabscheider durch azeotrope Destillation ausgekreist wird (9 Teile). Nach Einengen und Abkühlen des Reaktionsgemisches wird der Endstoff abfiltriert und

getrocknet. Es werden 139 Teile 2-Oxo-5,5-dimethyl-6-isopropyl-4-N-carbaminsäuremethoxyäthylester erhalten. Das entspricht einer Ausbeute von 97% der Theorie. Fp 143°C (Äthanol).

Beispiel 10

Analog Beispiel 8 werden 93 Teile 2-Oxo-4-hydroxy-5,5-dimethyl-6-isopropylhexahydropyrimidin zusammen mit 114,5 Teilen n-Dodecylcarbamat und 500 Teilen Xylol eine Stunde bei Rückflusstemperatur (138 bis 140°C) erwärmt, wobei das entstehende Reaktionswasser durch azeotrope Destillation über einen Wasserabscheider abgetrennt wird. Nach Einengen und Abkühlen wird der kristalline Endstoff abfiltriert und getrocknet. Es werden 192 Teile 2-Oxo-5,5-dimethyl-6-isopropyl-hexahydropyrimidyl-4-N-carbaminsäuredodecylester erhalten. Das entspricht einer Ausbeute von 97% der Theorie. Fp 126 bis 127°C (Aceton).

Beispiel 11

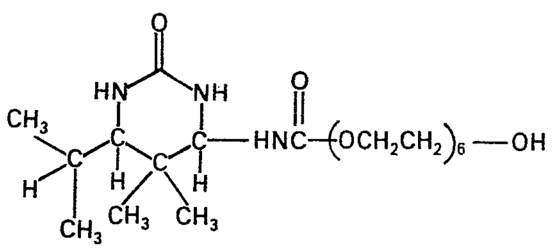

Das Gemisch von 93 Teilen 2-Oxo-4-hydroxy-5,5-dimethyl-6-isopropyl-hexahydropyrimidin und 162,5 Teilen Hexaäthylenglykolmonocarbamat ($HO-(CH_2CH_2-O)_6CONH_2$) in 500 Teilen Xylol wird analog Beispiel 8 45 Minuten bei einer Rückflusstemperatur von 134 bis 140°C erwärmt, wobei in dieser Zeit 9 Teile Wasser über den Wasserabscheider azeotrop abdestilliert werden. Nach Abdestillieren des Xylols und Trocknen des Endstoffs werden 249 Teile des 2-Oxo-5,5-dimethyl-6-isopropyl-hexahydropyrimidyl-N-carbaminsäurehexaäthylenglykolmonoesters erhalten. Das entspricht einer Ausbeute von 98 Prozent. Der Endstoff ist wasserklar und eine hochviskose Flüssigkeit.

Beispiel 12

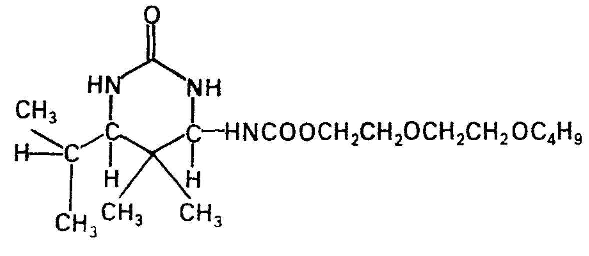

93 Teile 2-Oxo-4-hydroxy-5,5-dimethyl-6-isopropylhexahydropyrimidin, 102,5 Teile Butyldiäthylenglykolmonocarbamat und 500 Teile Xylol werden analog Beispiel 8 30 Minuten auf Rückflusstemperatur (132 bis 140°C) erwärmt. Das entstehende Reaktionswasser wird azeotrop abdestilliert. Nach Abkühlen wird der kristalline Endstoff abfiltriert und getrocknet, wobei 182 Teile 2-Oxo-5,5-dimethyl-6-isopropyl-hexahydropyrimidyl-4-N-carbaminsäureäthylenglykolmonoestermonobutyläther erhalten werden. Das entspricht einer Ausbeute von 98% der Theorie. Fp 108 bis 109°C (Aceton).

Beispiel 13

Analog Beispiel 8 wird das Gemisch aus 93 Teilen 2-Oxo-4-hydroxy-5,5-dimethyl-6-isopropylhexahydropyrimidin, 90,5 Teilen Phenylglykolacarbamat und 500 Teilen Xylol eine Stunde bei Rückflusstemperatur (135 bis 138°C) erwärmt. Das entstehende Reaktionswasser wird azeotrop durch Destillation abgetrennt. Nach Abdestillieren des Xylols und Trocknen des kristallinen Rückstandes werden 171 Teile 2-Oxo-5,5-dimethyl-6-isopropyl-hexahydropyrimidyl-4-N-carbaminsäurephenylglykolester erhalten. Das entspricht einer Ausbeute von 98% der Theorie. Fp 165 bis 167°C (Methanol).

**Patentanspruch**

Verfahren zur Herstellung von 5,5-disubstituierten 4-Carbamidohexahydropyrimidinen der Formel

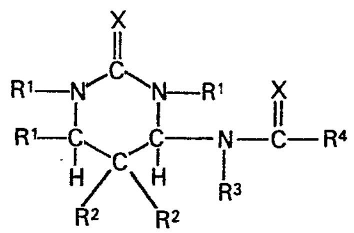

I,

worin die einzelnen Reste R¹, R² und R³ gleich oder verschieden sein können und jeweils einen aliphatischen, cycloaliphatischen, araliphatischen oder aromatischen Rest bedeuten, darüber hinaus auch die Reste R¹ und R³ jeweils ein Wasserstoffatom bezeichnen können, R⁴ den Rest 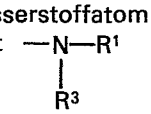

oder den Rest $-O-R^5$ bedeutet, $R^5$ einen aliphatischen Rest oder den Rest

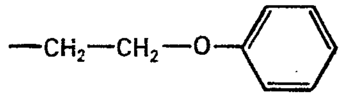

bezeichnet, die beiden Reste R³, sofern R⁴ den Rest 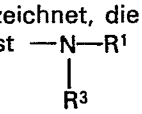

bezeichnet, auch zusammen mit der benachbarten Ureidogruppe für Glieder eines heterocyclischen Ringes stehen können, R³, sofern R⁴ den

Rest $-OR^5$ bedeutet, und $R^4$ zusammen mit dem benachbarten Carbamidorest auch für Glieder eines heterocyclischen Ringes stehen können, die einzelnen Reste X gleich oder verschieden sein können und jeweils ein Sauerstoffatom oder ein Schwefelatom bedeuten, durch Umsetzung von Hexahydropyrimidinen der Formel

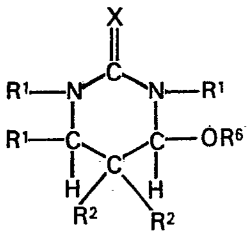

II,

worin $R^1$, $R^2$ und X die vorgenannte Bedeutung besitzen und $R^6$ ein Wasserstoffatom oder einen aliphatischen Rest bezeichnet, mit Carbamiden der Formel

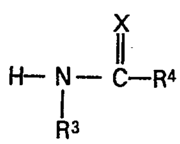

III,

worin $R^3$, $R^4$ und X die vorgenannte Bedeutung besitzen, dadurch gekennzeichnet, dass man ohne Säurezusatz bei einer Temperatur zwischen 100 und 150°C umsetzt.

**Claim**

A process for the preparation of 5,5-disubstituted 4-carbamidohexahydropyrimidines of the formula

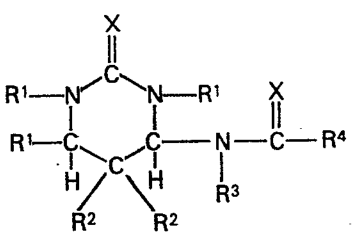

I,

where the individual radicals $R^1$, $R^2$ and $R^3$ may be identical or different and each is an aliphatic, cycloaliphatic, araliphatic or aromatic radical, the radicals $R^1$ and $R^3$ may also each be a hydrogen atom, $R^4$ is the radical 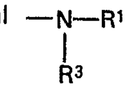

or the radical $-O-R^5$, $R^5$ is an aliphatic radical or the radical

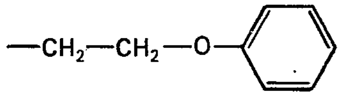

the two radicals $R^3$ can, if $R^4$ is the radical 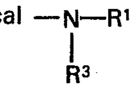

also be, together with the adjacent ureido group, members of a heterocyclic ring, and $R^3$, if $R^4$ is the radical $-OR^5$, and $R^4$ together with the adjacent carbamido radical can also be members of a heterocyclic ring, and the individual radicals X may be identical or different and each is an oxygen atom or a sulphur atom, by reacting hexahydropyrimidines of the formula

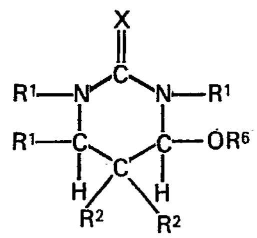

II,

where $R^1$, $R^2$ and X have the above meaning and $R^6$ is a hydrogen atom or an aliphatic radical, with carbamides of the formula

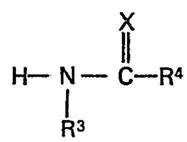

III,

where $R^3$, $R^4$ and X have the above meaning, characterised in that the reaction is carried out without addition of acid, at a temperature of from 100 to 150°C.

**Revendication**

Procédé de préparation de 4-carbamidohexahydropyrimidines disubstituées en position 5, de formule

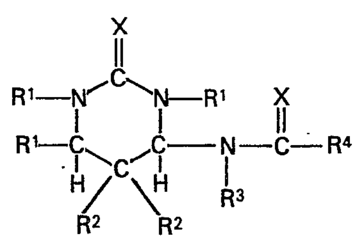

I,

dans laquelle les divers symboles $R^1$, $R^2$ et $R^3$, identiques ou différents, représentent chacun un reste aliphatique, cycloaliphatique, araliphatique ou aromatique, les symboles $R^1$ et $R^3$ pouvant en outre représenter chacun un atome d'hydrogène, $R^4$ représente le reste 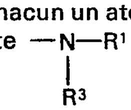

ou le reste -O-$R^5$, $R^5$ représente un reste aliphatique ou le

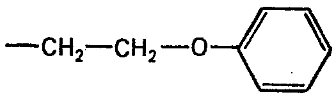

les deux symboles $R^3$ pouvant également représenter ensemble et avec le groupe uréido voisin, pour autant que $R^4$ ne représente pas le reste

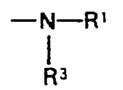

des membres d'un noyau hétérocyclique, $R^3$ et $R^4$ pouvant également former ensemble et avec le reste carbamido voisin, pour autant que $R^4$ ne représente pas le reste -O$R^5$, des membres d'un noyau hétérocyclique, les divers symboles X, ayant des significations identiques ou différentes, représentant chacun un atome d'oxygène ou un atome de soufre, par réaction d'hexahydropyrimidines de formule

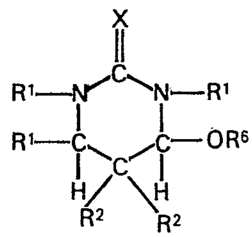

II,

dans laquelle $R^1$, $R^2$ et X ont les significations indiquées ci-dessus et $R^6$ représente un atome d'hydrogène ou un reste aliphatique, avec des carbamides de formule

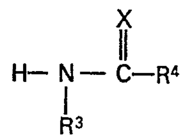

III,

dans laquelle $R^3$, $R^4$ et X ont les significations indiquées ci-dessus, caractérisé en ce que l'on fait réagir à une température de 100 à 150°C sans adjonction d'acide.